# EUROPÄISCHE PATENTSCHRIFT

(11) **EP 1 839 583 B1**
(45) Veröffentlichungstag und Bekanntmachung des Hinweises auf die Patenterteilung: **01.09.2010**
(21) Anmeldenummer: 07006432.4
(22) Anmeldetag: 28.03.2007
(51) Int. Cl.: A61B 17/02

(54) **Vorrichtung zum zeitweiligen Immobilisieren von Gewebe im Bereich eines pulsierend durchströmten Blutgefässes**
Device for temporary immobilisation of tissue near a pulsing blood vessel with blood flow
Dispositif d'immobilisation intermittente de tissu dans la zone d'un vaisseau sanguin passant de manière pulsée

(30) Priorität: 30.03.2006 DE 102006016003
(43) Veröffentlichungstag der Anmeldung: 03.10.2007
(73) Patentinhaber: Karl Storz GmbH & Co. KG, 78532 Tuttlingen (DE)
(72) Erfinder: Efinger, Andreas, 78604 Rietheim (DE); Hermle, Rainer, 78559 Gosheim (DE)
(74) Vertreter: Weller, Wolfgang

(56) Entgegenhaltungen:
- WO-A-98/40018
- US-A- 6 036 641
- US-A1- 2004 082 830

## Beschreibung

Die Erfindung betrifft eine Vorrichtung zum zeitweiligen Immobilisieren von Gewebe im Bereich eines pulsierend durchströmten Blutgefäßes, insbesondere eines Herzkranzgefäßes, während eines herz- und/oder thoraxchirurgischen Eingriffes, mit einem Fuß, der über einen Anlagebereich an das Gewebe anlegbar ist, wobei im Anlagebereich Ansaugöffnungen vorhanden sind, die mit einer Unterdruckquelle verbindbar sind.

Eine derartige Vorrichtung ist beispielsweise aus der WO 02/28287 A1 bekannt.

Solche Vorrichtungen werden bei herz- und/oder thoraxchirurgischen Eingriffen eingesetzt, bei denen am schlagenden Herzen Eingriffe an den Herzkranzgefäßen vorgenommen werden. Eine dieser Techniken ist die so genannte MIDCAB (Minimal Invasive Direct Coronar Artery Bypass)-Technik. Diese Technik erfolgt über einen Zwischenrippenraum.

Bei einer weiteren Operationstechnik wird das Brustbein der Länge nach durchgetrennt, und über so genannte Rippensperrer oder Retraktoren wird der Brustkorb seitlich aufgespreizt. Für einen besseren Zugang zum Herzen wird noch die eine Brustkorbhälfte, nämlich die linke, gegenüber der rechten Brustkorbhälfte angehoben. Nunmehr können Eingriffe am schlagenden Herzen durchgeführt werden, ohne dass das Herz mit einer Herz-Lungen-Maschine verbunden werden muss, so genannte OPCAB (Off Pump Coronary Artery Bypass)-Eingriffe.

Bei Eingriffen am schlagenden Herzen wird beispielsweise eine Brustbeinarterie, die zuvor entsprechend präpariert wurde, direkt an ein Gefäß des schlagenden Herzens angenäht, um bei einer Herzkranzgefäßverengung oder -verstopfung dadurch einen Bypass zu schaffen. Da, wie zuvor erwähnt, das Herz aber noch schlagend ist, also pulsierende Bewegungen durchführt, ist es sehr schwierig, an dieser Stelle einen Eingriff vorzunehmen.

Um das Gewebe im Bereich des Gefäßes, an dem der Bypass gelegt werden soll, zu immobilisieren oder zu stabilisieren, haben sich die eingangs erwähnten Vorrichtungen, die auch OPCAB-Stabilisatoren genannt werden, etabliert.

Derartige Vorrichtungen weisen einen Fuß auf, der beidseits und längsseits des Gefäßes des schlagenden Herzens an benachbartes Gewebe angelegt werden kann und dadurch diesen Bereich immobilisiert.

Je nach Ausgestaltung ist der Fuß gabelkopfartig mit zwei sich etwa parallel zueinander erstreckenden Zinken ausgebildet, die beidseits des Gefäßes angelegt werden können, oder er besteht, wie bei der eingangs erwähnten Druckschrift, aus zwei Einzelbauteilen, die jeweils an eine Seite des Gefäßes angelegt werden können. Der Fuß bzw. die Zinke ist mit einem Arm verbunden, der wiederum mit einem stationären Gerät verbunden ist, beispielsweise an einem zuvor erwähnten Rippensperrer angebracht ist. Somit kann der Fuß unter einem gewissen Druck an das Gewebe angelegt werden und immobilisiert dieses im Bereich links und rechts des Gefäßes, an dem der Eingriff vorgenommen werden muss.

Im Fuß sind Ansaugöffnungen vorgesehen, über die das Gewebe zusätzlich noch über einen Unterdruck an den Fuß angesaugt werden kann, so dass pulsierende Bewegungen in diesem Bereich kaum stattfinden können und der Operateur eine weitgehend ruhende, also immobilisierte, Operationsstelle vorfindet.

Aufgrund der Tatsache, dass solche Eingriffe an einem der wichtigsten Lebensorganen stattfindet, dem noch schlagenden Herzen, das allerdings aufgrund pathologischer Veränderungen stark in seiner Funktionsfähigkeit beeinträchtigt ist, besteht ein Bestreben, den Fuß an die anatomischen Anomalien in dem Anlagebereich möglichst gut anpassen zu können.

Daher sind in der eingangs erwähnten WO 02/28287 A1 und insbesondere in der WO 00/15119 A2 zahlreiche Verstellmöglichkeiten im Bereich des Fußes vorgesehen, so dass dieser mit möglichst vielen Freiheitsgraden verstellt werden kann. Dazu gehört ein Verdrehen, Verkippen und ein Verschwenken des Fußes bzw. der beiden Zinken relativ zueinander. Hierdurch ist es zwar möglich, an bestimmte Ausrichtungen des Gewebes anzupassen, letztendlich muss sich aber das Gewebe immer dem Fuß, der aus steifen Materialien hergestellt ist, anpassen.

In der WO 00/15119 A2 ist an einer Stelle erwähnt, den gesamten Fuß aus einem plastisch verformbaren Material herzustellen, so dass er vom Operateur an Ort und Stelle in eine bestimmte Ausrichtung gebogen werden kann. Allerdings muss sich auch dann das Gewebe diesem abgebogenen, beispielsweise stufenförmig gebogenen Fuß anpassen.

Aus der US 6,074,375 ist eine Vorrichtung zum Einbringen einer Flüssigkeit in den Herzmuskel bekannt. Die Flüssigkeit wird mit einer Art Spritze in den Muskel des schlagenden Herzens eingebracht. Um den Gewebebereich an der Einstichstelle zum Anzielen zu immobilisieren, ist ein zweibeiniger Saugfuß vorgesehen, der an den Herzmuskel angelegt werden kann und durch den ein Vakuum angelegt werden kann. Saugöffnungen am äußeren Ende der beiden Beine dienen zum Ansaugen des Gewebes. Die Beine weisen biegsame Abschnitte auf, um diese an die Kontur der Herzoberfläche anzupassen. Die Beine verbleiben in der verbogenen Ausrichtung. Eine weichelastische Schicht dient als Dichtung zwischen Muskeloberfläche und äußerem Ende mit den Saugöffnungen. Auch hier passt sich das Gewebe der Formgebung des Fußes an.

Aus der DE 20 2004 012 637 U1 ist ein Saugstabilisator mit einem Basiskörper und einer daran anbringbaren, relativ harten Saugleiste bekannt.

Es ist daher Aufgabe der vorliegenden Erfindung, eine Vorrichtung der eingangs erwähnten Art auszubilden, die ein atraumatischeres Immobilisieren des Gewebes ermöglicht.

Erfindungsgemäß wird die Aufgabe dadurch gelöst, dass der Fuß einen steifen Halter aufweist, der im Anlagebereich ein flexibles Element trägt, das die Ansaugöffnungen aufweist, wobei die Flexibilität derart ausgebildet ist, dass sich die Kontur des flexiblen Elements an die Kontur des Gewebes im Anlagebereich anpasst.

Die nunmehr vorgeschlagene Ausgestaltung ermöglicht es zum einen, über den steifen Halter eine ganz bestimmte räumliche Ausrichtung des Fußes gegenüber dem Gewebe zu bewerkstelligen, die räumlich unverrückbar ist und die auch dafür sorgt, dass ein gewisser Anpressdruck ausgeübt werden kann. Durch Vorsehen des flexiblen Elementes im Bereich des Anlagebereiches ist zum anderen die Möglichkeit geschaffen, dass sich im Anlagebereich im Bereich des flexiblen Elements der Fuß an die Formgebung des Gewebes in gewissem Maße anpassen kann, und nicht umgekehrt. Befindet sich beispielsweise in dem Anlagebereich ein Höcker im Gewebe, kann sich dieser in das flexible Element hineindrücken, so dass sich die Kontur des flexiblen Elements an die Kontur des Gewebes im Anlagebereich anpasst. Da in dem flexiblen Element die Ansaugöffnungen vorhanden sind, liegen die Ansaugöffnungen exakt angeschmiegt an dem Gewebebereich an, auch beispielsweise an gekrümmten Bereichen oder an dem zuvor erwähnten Höcker. Werden nun die Ansaugöffnungen mit Unterdruck beaufschlagt, wird das in dem Anlagebereich anliegende Gewebe lediglich zur zusätzlichen Halterung angesaugt, aber in seiner Gesamtheit nicht mehr verformt oder verändert.

Anders ausgedrückt, würde beispielsweise ein steifer geradliniger Halter an einen Gewebebereich mit dem zuvor erwähnten Höcker angelegt, müsste der Höcker in die Tiefen des Gewebes eingedrückt werden. Wenn das verhindert werden soll, würde das Gewebe, das ja im Bereich des Höckers näher oder stärker an dem Anlagebereich anliegt, mehr angesaugt als in anderen Bereichen, d.h. in diesen Bereichen würde beim Anlegen des Unterdruckes eine relativ starke Verformung stattfinden.

Dies könnte beispielsweise dazu führen, dass der Operateur zunächst den Fuß, ohne Beaufschlagung der Unterdruckquelle, aus seiner Sicht sehr gut ausgerichtet an dem Gewebe angelegt hat, nach dem Anlegen des Unterdruckes aber dann unerwünschte Lageveränderungen oder Gewebeverschiebungen stattfinden können.

Dies kann mit der Ausgestaltung mit dem flexiblen Element weitgehend deswegen ausgeschlossen werden, da sich Gewebe und flexibles Element im Anlagebereich aneinander anpassen, sich somit das flexible Element an das Gewebe anschmiegen kann bzw. umgekehrt. Der über die Ansaugöffnungen im flexiblen Element angelegte Unterdruck führt dann nicht mehr zu größeren Gewebeverschiebungen, sondern lediglich zu einer zusätzlich anhaftenden Fixierung des Gewebes am Fuß. Somit kann ein besonders atraumatisches Anlegen und Immobilisieren des Gewebes beispielsweise am schlagenden Herzen erzielt werden.

In einer weiteren Ausgestaltung der Erfindung ist das flexible Element lösbar am Halter angebracht.

Diese Maßnahme hat den Vorteil, dass je nach den anatomischen Gegebenheiten Elemente unterschiedlicher Flexibilität angebracht werden können.

In einer weiteren Ausgestaltung der Erfindung ist der Halter aus metallischem Material.

Diese Maßnahme hat den Vorteil, insbesondere mit der Ausgestaltung der lösbaren Anbringbarkeit des flexiblen Elements, dass der Halter für einen mehrfachen Einsatz geeignet ist, also nach einem Eingriff nach Abnehmen des flexiblen Elements entsprechend gereinigt und sterilisiert werden kann.

Dazu ist in einer weiteren Ausgestaltung vorgesehen, dass das flexible Element als Wegwerfteil für den Einmalgebrauch ausgebildet ist.

Diese Maßnahme hat den Vorteil, dass nach Durchführen einer Operation das flexible Element abgenommen und weggeworfen wird. Der Arm kann in diesem Zustand, also ohne das flexible Element, gereinigt und sterilisiert werden, und für einen nächsten Eingriff wird ein neues flexibles Element angesetzt.

In einer weiteren Ausgestaltung der Erfindung ist das flexible Element als schlauchförmiges Element ausgebildet.

Diese Maßnahme hat den Vorteil, dass die Schlauchgeometrie, unter Auswahl von entsprechenden Dimensionen und Materialien, eine hohe Variabilität bezüglich des Maßes der Flexibilität des Elementes erlaubt. Gleichzeitig kann über das schlauchförmige Element ein Unterdruck angelegt werden, um die zusätzliche Fixierung des Gewebes am schlauchförmigen Element zu erzielen.

In einer weiteren Ausgestaltung der Erfindung ist das schlauchförmige Element an einem Ende mit einem Saugstutzen des Halters verbindbar.

Diese Maßnahme hat den Vorteil, dass das zuvor erwähnte Beaufschlagen mit Unterdruck einfach möglich ist, indem nämlich das schlauchförmige Element an einem entsprechenden Saugstutzen des Halters aufgeschoben wird.

In einer weiteren Ausgestaltung der Erfindung kommt das flexible Element im Anlagebereich im Abstand zum Halter zum Liegen.

Diese Maßnahme hat den Vorteil, dass das flexible Element seitlich ausweichen oder ausbauchen kann, um sich beispielsweise extremen Krümmungen oder sonstigen Anomalien im Bereich des Gewebes anpassen zu können. Die Tatsache, dass das flexible Element an einem steifen Halter angebracht ist, begrenzt jedoch dieses Ausweichen, so dass der erwünschte Anpressdruck ausgeübt werden kann.

In einer weiteren Ausgestaltung der Erfindung ist der Halter als Streifen ausgebildet, von dessen Anlageseite zumindest ein Halteelement vorsteht, an das das flexible Element anbringbar ist.

Diese Maßnahme hat den Vorteil, dass an der Anlageseite des Streifens relativ viel Platz zur Verfügung steht, um die Haltevorrichtungen für das flexible Element vorzusehen, an die das flexible Element je nach Ausgestaltung lösbar oder unlösbar anbringbar ist.

In einer weiteren Ausgestaltung der Erfindung ist das schlauchförmige Element als Schlauchstück ausgebildet, das an einem Ende auf einen Saugstutzen aufschiebbar und am anderen Ende auf einen Haltezapfen aufschiebbar ist.

Diese Maßnahme hat den Vorteil, dass das schlauchförmige Element besonders kostengünstig herzustellen ist, besonders einfach zu handhaben ist und aufgrund seiner Geometrie ein besonders atraumatisches Anlegen erlaubt.

Das entsprechende Schlauchstück, das beispielsweise als Meterware mit Solltrennstellen oder auch als Einzelstück vorrätig ist, braucht lediglich auf die beiden Stutzen aufgeschoben zu werden, so dass die Vorrichtung einsatzbereit ist.

In einer weiteren Ausgestaltung der Erfindung ist der Fuß als Gabelkopf mit zwei Zinken ausgebildet, wobei jede Zinke als steifer Halter mit einem daran angebrachten flexiblen Element ausgebildet ist.

Diese Maßnahme hat den Vorteil, dass durch ein einziges Element der Fuß beidseits des zu immobilisierenden Gefäßes in einem Vorgang angelegt werden kann und der Fuß auf beiden Seiten mit einem flexiblen Element ausgestattet ist, so dass der gesamte Fuß besonders atraumatisch an das Gewebe anlegbar ist.

In einer weiteren Ausgestaltung der Erfindung sind in dem Mantel des Schlauchstückes die Ansaugöffnungen vorgesehen.

Diese Maßnahme hat den Vorteil, dass dies maschinell sehr einfach bewerkstelligt ist und das Schlauchstück als vorbereitetes, gegebenenfalls steril verpacktes Austauschteil zur Verfügung steht.

In einer weiteren Ausgestaltung der Erfindung ist der Fuß an einem in allen Raumrichtungen biegbaren und in dieser Biegestellung feststellbaren Arm angebracht.

Diese an sich bekannte Maßnahme hat den Vorteil, dass der Fuß vom Operateur in eine für ihn ergonomisch günstige und für die Operation gut zugänglichen Stellung über den Arm angelegt und dann in dieser Stellung festgestellt werden kann.

In einer weiteren Ausgestaltung der Erfindung ist der Arm an dem dem Fuß gegenüberliegenden Ende mit einer Halterung versehen, über die der Arm an einem Retraktor befestigbar ist.

Diese Maßnahme hat den Vorteil, dass die Vorrichtung an ein Bauteil anbringbar ist, das ohnehin bei der Operation vorhanden ist, nämlich den Retraktor bzw. den Rippenspreizer, der den Brustkorb spreizt. Dieses Bauteil ist relativ ortsfest und dient als Widerlager für den Arm, so dass der Fuß durch den Operateur sehr einfach in eine bestimmte raum- und ortsfeste Relativstellung zum Retraktor gebracht werden kann, wobei der Fuß dann zur Anlage an dem Gewebe kommt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert.
Es zeigen:
- Fig. 1: eine perspektivische Darstellung einer erfindungsgemäßen Vorrichtung, die an einer Schiene eines Retraktors angebracht ist,
- Fig. 2: eine perspektivische Detailansicht eines Fußes der Vorrichtung von Fig. 1 in einer teilweisen Explosionsdarstellung, also mit noch nicht angelegtem bzw. abgenommenem flexiblen Element,
- Fig. 3: eine stark vergrößerte perspektivische Detailansicht des Fußes von Fig. 1,
- Fig. 4: einen Schnitt längs der Linie IV-IV in Fig. 3 mit angeschlossener Vakuumleitung, und
- Fig. 5: einen der Fig. 4 vergleichbaren Schnitt, wobei die Anpassung der Kontur des flexiblen Elements an die Kontur des Gewebes ersichtlich ist.

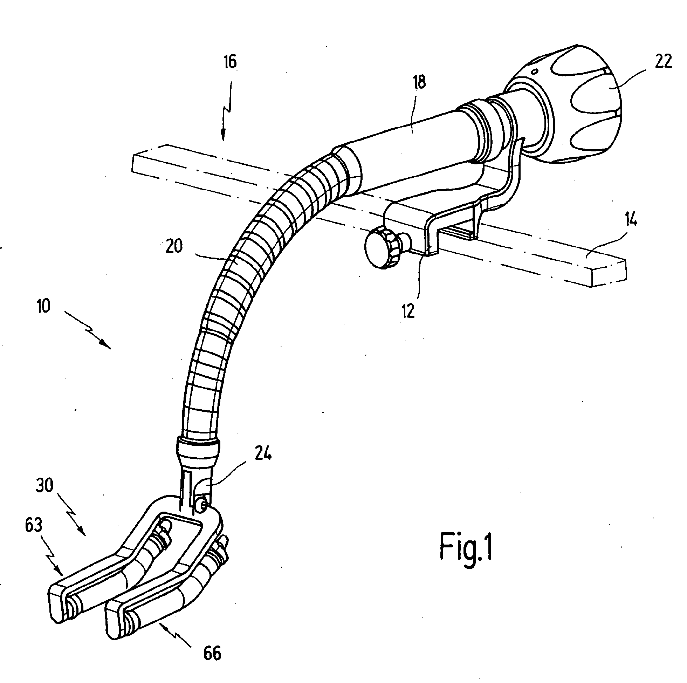

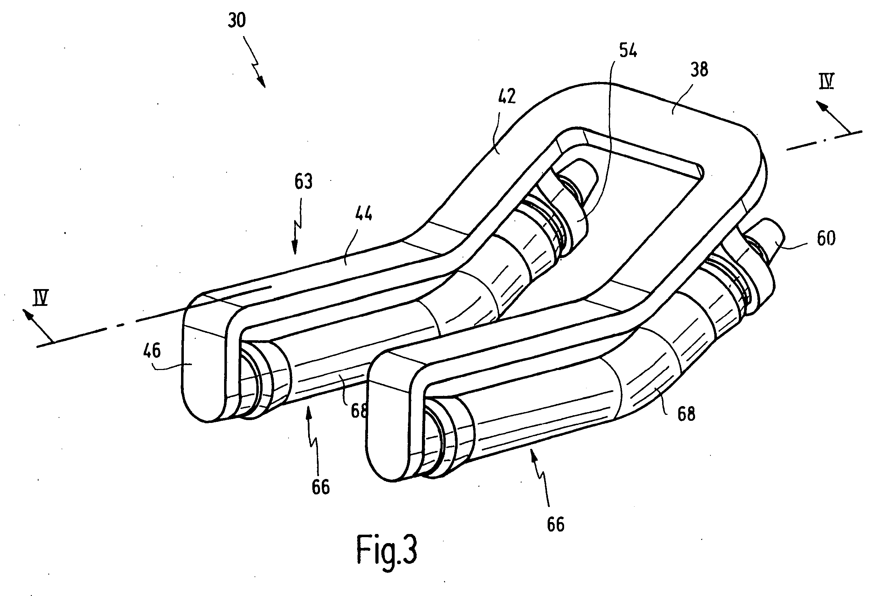

Eine in den Fig. 1 bis 5 dargestellte erfindungsgemäße Vorrichtung ist in ihrer Gesamtheit mit der Bezugsziffer 10 versehen.

Die Vorrichtung 10 weist an einem Ende eine Halterung 12 auf, über die die Vorrichtung 10 an eine Schiene 14 eines Retraktors 16 bzw. eines Rippenspreizers angebracht werden kann.

In dieser Ausgestaltung ist somit die Vorrichtung 10 ein Funktionselement eines Retraktors.

Von der Halterung 12 springt ein Arm 18 vor, der als ein so genannter Leyla-Arm 20 ausgebildet ist. Ein solcher Leyla-Arm 20 zeichnet sich dadurch aus, dass er in sämtliche Raumrichtungen gebogen werden und in einer solchen gebogenen Stellung arretiert werden kann. Dazu besteht ein solcher Leyla-Arm 20 aus einzelnen gelenkartigen Elementen, die diese Verbiegungen ermöglichen. Zum Arretieren ist mittig durchgehend ein Ziehdraht vorhanden, der mit einem Stellknopf 22 verbunden ist. Wie erwähnt, kann bei gelöstem Stellknopf 22 der Arm 18 in allen Raumrichtungen verändert werden. Durch Festdrehen des Stellknopfes bleibt der Arm, beispielsweise in der in Fig. 1 dargestellten gebogenen Stellung, unverrückbar stehen.

An seinem dem Stellknopf 22 gegenüberliegenden Ende ist der Arm über ein Gelenk 24 mit einem Fuß 30 verbunden.

Der Fuß 30 ist als ein Gabelkopf 32 ausgebildet, der zwei parallel zueinander verlaufende Zinken 34 und 36 aufweist, die über einen Querbügel 38 miteinander verbunden sind. Der Gabelkopf 32 ist aus einem Streifen 40 aus einem metallischen Material hergestellt.

Jede der beiden Zinken 34 und 36 weist, ausgehend vom Querbügel 38, einen ersten Abschnitt 42 auf, der über eine Biegung in einen zweiten Abschnitt 44 übergeht, dessen äußerstes Ende etwa rechtwinklig in einen abgebogenen Endabschnitt 46 übergeht.

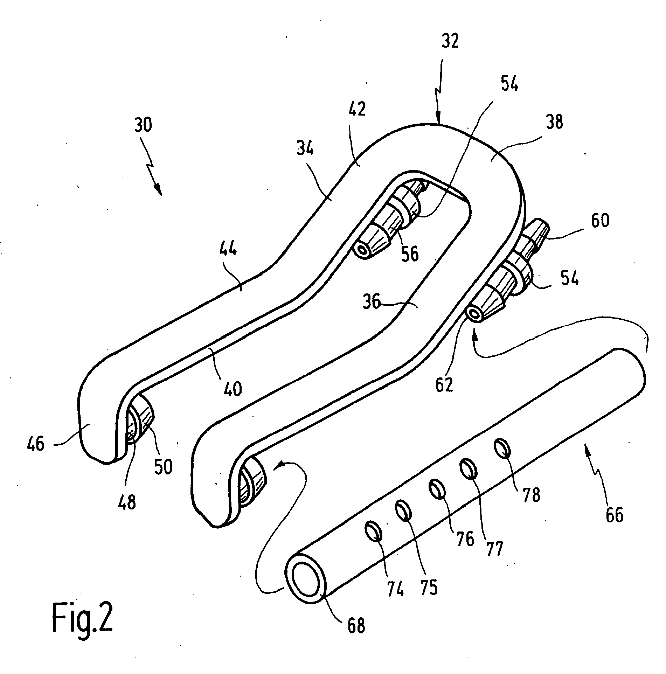

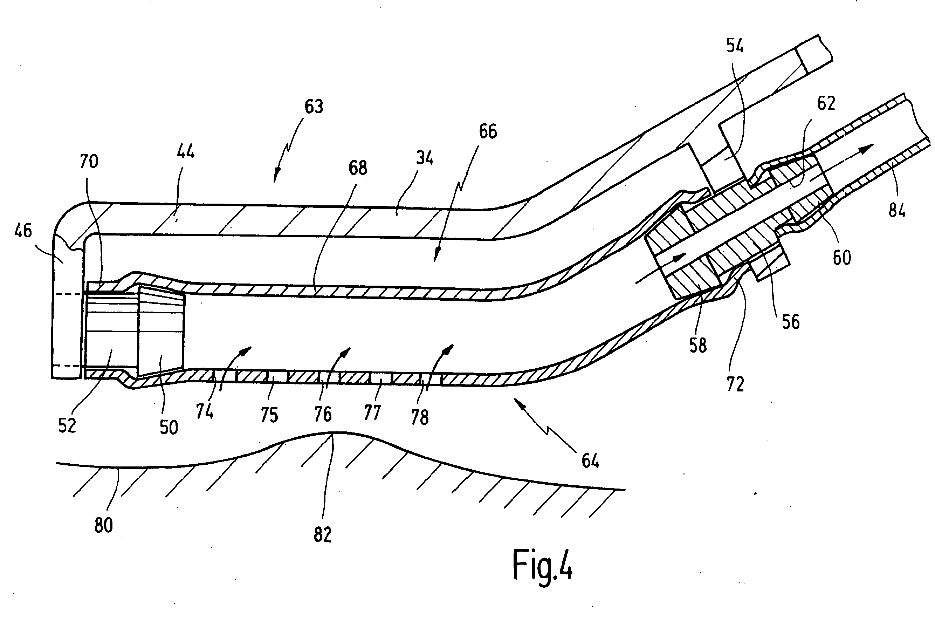

Von der Innenseite 48 des abgebogenen Abschnittes 46 steht ein konischer Zapfen 50 vor (siehe insbesondere Fig. 2 und 4).

Der konische Zapfen 50 weist einen Hals 52 auf, der in einer hier nicht näher dargestellten Öffnung im abgebogenen Abschnitt 46 aufgenommen ist.

Von der Unterseite jeder Zinke 34 und 36 steht im Bereich nach dem Querbügel 38 jeweils eine Öse 54 vor, in die ein Saugstutzen 56 montiert ist.

Der Saugstutzen 56 weist einen ersten Stutzen 58 auf, der konisch verläuft und der dem konischen Zapfen 50 zugewandt ist.

Auf der gegenüberliegenden Seite der Öse 54 steht ein zweiter Stutzen 60 vor. Im Saugstutzen 56 mittig durchgehend verläuft ein Saugkanal 62.

Auf den konischen Zapfen 50 einerseits bzw. auf den ersten Stutzen 58 andererseits kann ein flexibles Element 66 in Form eines Schlauchstückes 58 aufgeschoben werden.

Aus der Schnittdarstellung von Fig. 4 ist ersichtlich, dass ein Ende 70 des Schlauchstückes 68 vollständig über den konischen Zapfen 50 und dessen Hals 52 geschoben ist, dort also festsitzend aufgenommen ist.

Aus der Schnittdarstellung von Fig. 4 ist ersichtlich, dass durch den Übergang von dem konischen Zapfen 50 zu dem zylindrischen Hals 52 eine Hinterschneidung geschaffen ist, über die das Ende 70 des Schlauchstückes 68 unter Aufweitung geschoben werden muss und dadurch ein Abziehen gehindert wird.

Entsprechendes gilt für das gegenüberliegende Ende 72 des Schlauchstückes 68. Dies ist auf den ersten Stutzen 58 des Saugstutzens 56 entsprechend unverlierbar aufgeschoben.

Insbesondere aus der Schnittdarstellung von Fig. 4 ist ersichtlich, dass jede der Zinken 34 bzw. 36 im Zusammenhang mit dem konischen Zapfen 50 bzw. dem ersten Stutzen 58 als ein steifer Halter 63 ausgebildet ist, der als Halter für das flexible Element 66 dient.

Das Schlauchstück 68 besteht aus einem Siliconschlauch, in dessen Wand auf einer Seite fünf Ansaugöffnungen 74 bis 78 ausgespart sind (siehe insbesondere Fig. 2).

Das Schlauchstück 68 ist so gewählt, dass es in dem Abschnitt zwischen Zapfen 50 und Stutzen 58 eine Flexibilität aufweist, dennoch aber so formstabil ist, dass es bei einem im Inneren des Schlauchstückes 68 angelegten Unterdruck nicht kollabiert.

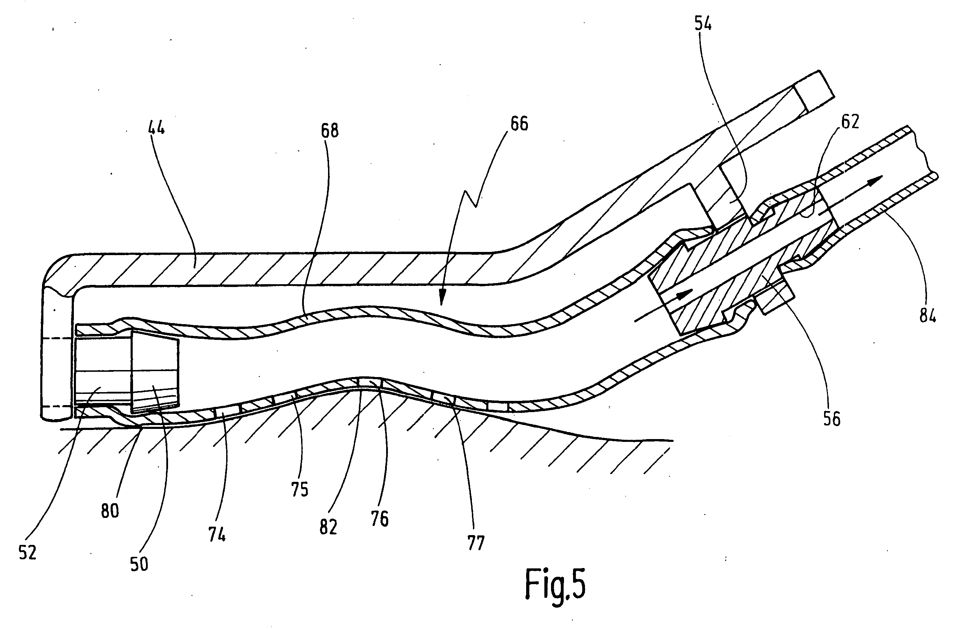

Wie aus Fig. 5 zu erkennen, ist auf den zweiten Stutzen 60 eine Vakuumleitung 84 aufgeschoben, die mit einer Unterdruckquelle verbunden ist.

Beim praktischen Einsatz wird die Vorrichtung 10 beispielsweise an ein Herzkranzgefäß so angelegt, dass sich ein Abschnitt dieses Gefäßes parallel und zwischen den beiden Zinken 34 und 36 erstreckt. Dazu wird die Vorrichtung 10 bzw. deren Fuß 30 in einem Anlagebereich 64, der sich etwa über die Länge des Schlauchstückes 68 erstreckt, an ein entsprechendes Gewebe 80 beidseits des zwischen den Zinken 34 und 36 liegenden Blutgefäßes angelegt.

Ist beispielsweise in diesem Bereich ein Höcker 82 vorhanden, kann sich das flexible Element 66 aufgrund der Flexibilität des Schlauchstückes 68 an die Krümmung des Höckers 82 in einem bestimmten Maß anpassen. Dadurch legen sich dann auch die fünf Ansaugöffnungen 74, 75, 76, 77 und 78 gleichförmig anschmiegend an das Gewebe 80 an. Es ist aus Fig. 4 zu erkennen, dass der Höcker 82 zunächst nur mit der Ansaugöffnung 76 in Berührung treten wird, die beidseits gelegenen Ansaugöffnungen 75 und 77 schon in einem gewissen Abstand vom Gewebe 80 zum Liegen kommen würden, und die endseitigen Ansaugöffnungen 74 und 78 noch in einem erheblichen Abstand zum Liegen kämen.

Aufgrund der Flexibilität des Schlauchstückes 68 kann sich dieses flexible Schlauchstück 68 atraumatisch an die Geometrie des Gewebes 80 anpassen, wie das aus Fig. 5 ersichtlich ist.

Durch den steifen Halter 63 kann aber durch das Schlauchstück 68 dennoch ein ausreichender Anpressdruck ausgeübt werden, um das Gewebe 80 in diesem Bereich zu immobilisieren.

Durch Anlegen eines Unterdruckes über die Vakuumleitung 84 kann das Gewebe 80 noch zusätzlich an dem Schlauchstück 68 fixiert und damit auch gleichzeitig weiter immobilisiert werden, wobei dies ebenfalls atraumatisch erfolgt, da sich vorher die Schlauchbereiche mit den Ansaugöffnungen 74 bis 78 bereits an das Gewebe 80 angeschmiegt haben.

Nach dem Eingriff braucht das Schlauchstück 68 lediglich von dem Zapfen 50 bzw. dem Stutzen 58 abgezogen zu werden und kann verworfen werden, so dass dann der Fuß 30 bzw. dessen metallische Bauteile einer Reinigung und Sterilisierung unterzogen werden können.

Für einen weiteren Eingriff wird dann jeweils ein neues Schlauchstück 68 an die Unterseite jeder Zinke 34 und 36 angebracht, so dass die Vorrichtung 10 für einen erneuten Eingriff bereit ist.

## Patentansprüche

1. Vorrichtung zum zeitweiligen Immobilisieren von Gewebe (80) im Bereich eines pulsierend durchströmten Blutgefäßes, insbesondere eines Herzkranzgefäßes, während eines herz- und/oder thoraxchirurgischen Eingriffs,
mit einem Fuß (30), der über einen Anlagebereich (64) an das Gewebe (80) anlegbar ist, wobei im Anlagebereich (64) Ansaugöffnungen (74 bis 78) vorhanden sind, die mit einer Unterdruckquelle verbindbar sind,
**dadurch gekennzeichnet, dass**
der Fuß (30) einen steifen Halter (63) aufweist, der im Anlagebereich (64) ein flexibles Element (66) trägt, das die Ansaugöffnungen (74-78) aufweist, wobei die Flexibilität derart ausgebildet ist, dass sich die Kontur des flexiblen Elements (66) an die Kontur des Gewebes (80) im Anlagebereich (64) anpasst.

2. Vorrichtung nach Anspruch 1, **dadurch gekennzeichnet, dass** das flexible Element (66) lösbar am Halter (63) angebracht ist.

3. Vorrichtung nach Anspruch 1 oder 2, **dadurch gekennzeichnet, dass** der Halter (63) aus metallischem Material hergestellt ist.

4. Vorrichtung nach einem der Ansprüche 1 bis 3, **dadurch gekennzeichnet, dass** das flexible Element (66) als Wegwerfteil für den Einmalgebrauch ausgebildet ist.

5. Vorrichtung nach einem der Ansprüche 1 bis 4, **dadurch gekennzeichnet, dass** das flexible Element als schlauchförmiges Element (66) ausgebildet ist.

6. Vorrichtung nach Anspruch 5, **dadurch gekennzeichnet, dass** das schlauchförmige Element (66) an einem Ende (72) mit einem Saugstutzen (56) des Halters (63) verbindbar ist.

7. Vorrichtung nach einem der Ansprüche 1 bis 6, **dadurch gekennzeichnet, dass** das flexible Element (66) im Anlagebereich (64) im Abstand zum Halter (63) zum Liegen kommt.

8. Vorrichtung nach einem der Ansprüche 1 bis 7, **dadurch gekennzeichnet, dass** der Halter (63) als Streifen (40) ausgebildet ist, von dessen Anlageseite zumindest ein Halteelement (50, 58) vorsteht, an das das flexible Element (66) anbringbar ist.

9. Vorrichtung nach einem der Ansprüche 1 bis 8, **dadurch gekennzeichnet, dass** das schlauchförmige Element (66) als Schlauchstück (68) ausgebildet ist, das an einem Ende (72) auf einen Saugstutzen (56) aufschiebbar ist und am anderen Ende (70) auf einen Haltezapfen (50) aufschiebbar ist.

10. Vorrichtung nach einem der Ansprüche 1 bis 9, **dadurch gekennzeichnet, dass** der Fuß (30) einen Gabelkopf (32) mit zwei Zinken (34, 36) aufweist, wobei jede Zinke (34, 36) als steifer Halter (63) mit einem daran angebrachten flexiblen Element (66) ausgebildet ist.

11. Vorrichtung nach Anspruch 9 oder 10, **dadurch gekennzeichnet, dass** in einem Mantel des Schlauchstückes (68) die Ansaugöffnungen (74-78) vorgesehen sind.

12. Vorrichtung nach einem der Ansprüche 1 bis 11, **dadurch gekennzeichnet, dass** der Fuß (30) an einem in allen Raumrichtungen biegbaren und in diesen Biegestellungen feststellbaren Arm (18) angebracht ist.

13. Vorrichtung nach Anspruch 12, **dadurch gekennzeichnet, dass** der Arm (18) an dem dem Fuß (30) gegenüberliegenden Ende mit einer Halterung (12) versehen ist, über die der Arm (18) an einem Retraktor (16) befestigbar ist.

## Claims

1. Device for temporarily immobilizing tissue (80) in the area of a pulsating flowed-through blood vessel, in particular a coronary vessel, during a cardiac and/or thoracic surgical intervention, with a foot (30) which can be placed onto the tissue (80) via a contact area (64), the contact area (64) having suction openings (74 to 78) that can be connected to an underpressure source, **characterized in that** the foot (30) comprises a rigid holder (63) which, in the contact area (64), supports a flexible element (66) that includes the suction openings (74-78), wherein the flexibility is **in that** the contour of the flexible element (66) adapts to the contour of the tissue (80) in the contact area (64).

2. Device of Claim 1, **characterized in that** the flexible element (66) is mounted releasably on the holder (63).

3. Device of Claims 1 or 2, **characterized in that** the holder (63) is made of metal material.

4. Device of anyone of Claims 1 to 3, **characterized in that** the flexible element (66) is designed as a disposable part for one-off use.

5. Device of anyone of Claims 1 to 4, **characterized in that** the flexible element is designed as a tubular element (66).

6. Device of Claim 5, **characterized in that** the tubular element (66) can be connected at one end (72) to a suction connector piece (56) of the holder (63).

7. Device of anyone of Claims 1 to 6, **characterized in that** the flexible element (66) in the contact area (64) comes to lie at a distance from the holder (63).

8. Device of anyone of Claims 1 to 7, **characterized in that** the holder (63) is designed as a strip (40) from whose contact face at least one retaining element (50, 58) protrudes onto which the flexible element (66) can be mounted.

9. Device of anyone of Claims 1 to 8, **characterized in that** the tubular element (66) is designed as a hose piece (68) which at one end (72) can be pushed onto a suction connector piece (56) and at the other end (70) can be pushed onto a retaining plug (50).

10. Device of anyone of Claims 1 to 9, **characterized in that** the foot (30) has a fork head (32) with two prongs (34, 36), each prong (34, 36) being designed as a rigid holder (63) with a flexible element (66) mounted on it.

11. Device of Claims 9 or 10, **characterized in that** the suction openings (74-78) are provided in a jacket of the hose piece (68).

12. Device of anyone of Claims 1 to 11, **characterized in that** the foot (30) is mounted on an arm (18) which can bend in all spatial directions and can be fixed in these bend positions.

13. Device of Claim 12, **characterized in that**, at the end remote from the foot (30), the arm (18) is provided with a support fixture (12) via which the arm (18) can be secured on a retractor (16).

## Revendications

1. Dispositif d'immobilisation temporaire de tissu (80) dans la région d'un vaisseau sanguin parcouru par un écoulement pulsatoire, en particulier d'un vaisseau coronaire, au cours d'une intervention chirurgicale cardiaque et/ou thoracique, comprenant une embase (30) pouvant être mise en applique contre le tissu (80) par l'intermédiaire d'une zone de contact (64), sachant que des orifices d'aspiration (74 à 78), présents dans ladite zone de contact (64), peuvent être raccordés à une source de dépression,
**caractérisé par le fait que**
ladite embase (30) présente un support rigide (63) portant, dans la zone de contact (64), un élément flexible (66) qui est percé desdits orifices d'aspiration (74-78), la flexibilité étant étudiée de telle sorte que le profil dudit élément flexible (66) s'adapte au profil du tissu (80) dans ladite zone de contact (64).

2. Dispositif selon la revendication 1, **caractérisé par le fait que** l'élément flexible (66) est installé sur le support (63) de manière dissociable.

3. Dispositif selon la revendication 1 ou 2, **caractérisé par le fait que** le support (63) est fabriqué en un matériau métallique.

4. Dispositif selon l'une des revendications 1 à 3, **caractérisé par le fait que** l'élément flexible (66) est réalisé sous la forme d'une pièce jetable, à usage unique.

5. Dispositif selon l'une des revendications 1 à 4, **caractérisé par le fait que** l'élément flexible est réalisé comme un élément (66) en forme de tuyau souple.

6. Dispositif selon la revendication 5, **caractérisé par le fait que** l'élément (66) en forme de tuyau souple peut être raccordé, par une extrémité (72), à un embout d'aspiration (56) du support (63).

7. Dispositif selon l'une des revendications 1 à 6, **caractérisé par le fait que** l'élément flexible (66) vient en applique, dans la zone de contact (64), à distance du support (63).

8. Dispositif selon l'une des revendications 1 à 7, **caractérisé par le fait que** le support (63) est réalisé sous la forme d'une barrette (40), au-delà de la face de contact de laquelle fait saillie au moins un élément d'arrêt (50, 58) sur lequel l'élément flexible (66) peut être mis en place.

9. Dispositif selon l'une des revendications 1 à 8, **caractérisé par le fait que** l'élément (66) en forme de tuyau souple se présente comme un segment de tuyau souple (68) pouvant être enfilé sur un embout d'aspiration (56), par une extrémité (72), et sur un mamelon d'arrêt (50) par l'autre extrémité (70).

10. Dispositif selon l'une des revendications 1 à 9, **caractérisé par le fait que** l'embase (30) offre une tête fourchue (32) munie de deux fourchons (34, 36), chaque fourchon (34, 36) étant réalisé sous la forme d'un support rigide (63) sur lequel un élément flexible (66) est implanté.

11. Dispositif selon la revendication 9 ou 10, **caractérisé par le fait que** les orifices d'aspiration (74-78) sont prévus dans une enveloppe du segment de tuyau souple (68).

12. Dispositif selon l'une des revendications 1 à 11, **caractérisé par le fait que** l'embase (30) est installée sur un bras (18) pouvant être fléchi dans toutes les directions spatiales, et pouvant être bloqué à demeure dans ces positions prises par flexion.

13. Dispositif selon la revendication 12, **caractérisé par le fait que** le bras (18) est pourvu, à l'extrémité pointant à l'opposé de l'embase (30), d'une monture de blocage (12) par l'intermédiaire de laquelle ledit bras (18) peut être fixé à un rétracteur (16).
